# EUROPEAN PATENT APPLICATION

(11) **EP 3 525 211 A1**
(43) Date of publication of application: **14.08.2019**
(21) Application number: 19155854.3
(22) Date of filing: 07.02.2019
(51) Int. Cl.: G16H 10/40, G16H 15/00

(54) **TEST ORDER PROCESSING APPARATUS, COMPUTER PROGRAM THEREFOR AND TEST SYSTEM**

(30) Priority: 07.02.2018 JP 2018020567
(71) Applicant: HORIBA, Ltd., Kyoto 601-8510 (JP)
(72) Inventor: DEJIMA, Yutaka, Kyoto, 601-8510 (JP); NISHIMORI, Masashi, Kyoto, 601-8510 (JP); SETO, Yoshinori, Kyoto, 601-8510 (JP)
(74) Representative: Isarpatent

(57) **Abstract**

According to the test order processing apparatus of the present invention, a test apparatus corresponding to a particular test order and a test item contained therein are linked and displayed on a display device, the test apparatus that should perform the test and a test order are selected by the user or automatically selected, and the test results received from the test apparatus are linked to the test order linked to the test apparatus based on the selection sequence.

## Description

### FIELD OF THE INVENTION

The present invention relates to a test order processing apparatus for linking a test item contained in a test order and a test result by a test apparatus, a computer program for constituting the test order processing apparatus with a computer, and a test system using the test order processing apparatus.

### BACKGROUND OF THE INVENTION

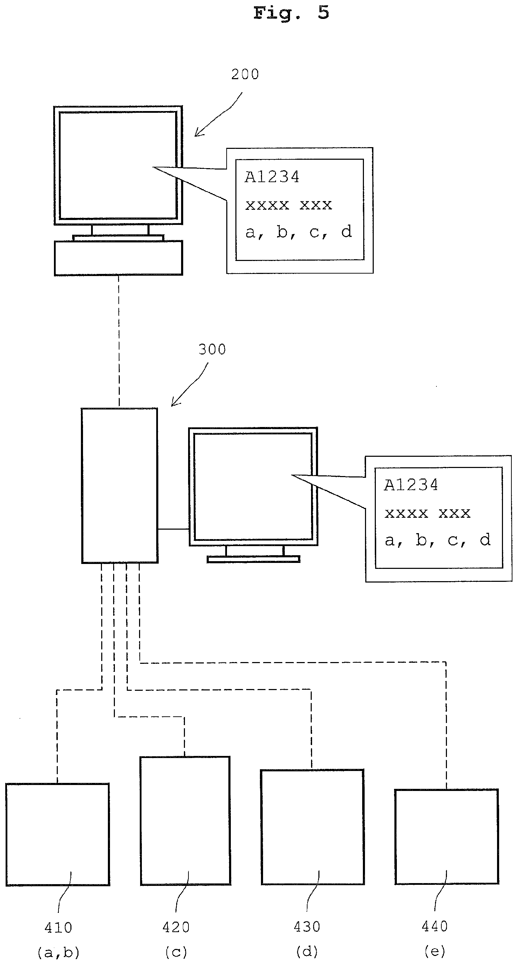

In recent years, many medical institutions introduce management systems using computers, and an increasing number of medical institutions introduce electronic medical records (medical charts formed on computer) instead of paper medical charts (medical examination records) hand-written by doctors. Between the doctor side and the test department, an on-line system for data communication between the test order and the test results between the computers has also been introduced (e.g., patent document 1 and the like). In such on-line system, as simply exemplified in Fig. 5, a test order is created on the computer 200 on the doctor side to request the test department for the necessary test, the test order is sent to the computer 300 in the test department, the test department performs various tests according to the test order, and the test results are sent back to the computer 200 on the doctor side from the computer 300 as a report for the test order.

A test order contains at least a test order ID which is information identifying the test order itself and test items necessary for diagnosis. In the test order of Fig. 5, as a simple example, test order ID (A1234), full name (xxxx xxx) as identifying information of the test subject (test subject ID), and test items a, b, c, d requesting specimen testing are contained.

On the other hand, for automatically performing tests (analysis and measurement) with respect to given test items of specimen (blood, urine, feces and the like) collected from a test subject, various test apparatuses have been introduced into test department (e.g., patent document 2 and the like). In many test apparatuses, not only a test with respect to a single test item but also tests with respect to multiple test items can be performed. In the test department, apparatuses capable of performing a test with respect to one or more test items contained in a test order (hereinafter to be also referred to as test apparatus suitable for test item) are provided to respond to all test items in test orders by combining plural test apparatuses. For example, in Fig. 5, four test apparatuses (410 (for the test items a, b), 420 (for the test item c), 430 (for the test item d), and 440 (for the test item e)) are provided, and test apparatuses (410, 420, 430) are selected to respond to the test items (a, b, c, d) in the test order.

Various test apparatuses provided in the test department each include a computer as a control part and data communication is possible with outside computers.

In the embodiment described in patent document 2, as shown in Fig. 5, the computer 300 in the test department functions as a test order processing apparatus that mediates between the computer 200 on the doctor side and various test apparatuses. A computer program that makes the computer 300 function as such test order processing apparatus is commercially available as an electronic medical records linkage software. The computer 300 that functions as a test order processing apparatus automatically selects a test apparatus corresponding to the test items in the test order, and sends the test order together with test order ID and test subject ID to the test apparatus. Each test apparatus that received the test order displays the test order ID and the test subject ID on each display device. A test staff (clinical laboratory technician, nurse etc.) sets a specimen accorded with a test subject ID on the test apparatus and starts the test. The test apparatus sends back the test results to computer 300 together with test order ID and test subject ID, and the computer 300 integrates the test results sent from respective test apparatuses and sends them back to computer 200 on the doctor-side as a test report to the test order.
[patent document 1]WO 2017-073567
[patent document 2]JP-A-2014-215210

The present inventors have studied in more detail the processing function of the aforementioned conventional test order processing apparatuses and newly found that the following problem is present.

The sending and receiving of test order ID and the like between the aforementioned test order processing apparatus (computer) and each test apparatus becomes available when the control part (computer) of the test apparatus becomes operative in linkage with the function of the test order processing apparatus. Some of the test apparatuses do not have such function (hereinafter to be also referred to as "test order ID send/receive non-compliant test apparatus"). For example, some of them do not have the function of receiving commands from an outside computer or the function of displaying test order ID but can simply output the test result data from output interface to an outside computer.

For example, when the test apparatus 440 in Fig. 5 is a test apparatus of the aforementioned test order ID send/receive non-compliant, a test staff selects the test apparatus 440 according to test item e contained in the test order, sets a specimen on the test apparatus and performs the test. The test apparatus 440 sends the data of the test results to computer (test order processing apparatus) 300. In this case, the test staff needs to perform a task of linking the test results from the test apparatus 440 to the truly corresponding test order on the computer 300 (hereinafter to be also referred to as linking work). The aforementioned linking work when the test apparatus is test order ID send/receive non-compliant varies depending on the program for test order processing by computer 300. For example, in computer 300 and the like, an operation of inputting and linking the test order ID of the truly corresponding test order to the test results, and an operation of linking by placing the data of the test results to a report folder linked to the truly corresponding test order are considered.

Such conventional linking work requires troublesome input of test order ID, and may make a mistake in inputting character strings of the test order ID or linking mistakes (linking test results to other test order). In addition, such linking work can be performed only after the test results are received from the test order ID send/receive non-compliant test apparatus. Therefore, the linking work sometimes needs to wait until the test apparatus performs the test and sends the test result data.

The problem newly found by the present inventors could occur in linking the test results from the test order ID send/receive non-compliant test apparatus to the truly corresponding test order ID.

An object of the present invention is to solve the above-mentioned problems and facilitate the work of linking the data of the test results sent from the test order ID send/receive non-compliant test apparatus to the truly corresponding test order.

### SUMMARY OF THE INVENTION

The main constitution of the present invention is as follows.
[1] A test order processing apparatus for linking a test result sent from a test apparatus to a test order corresponding to the test result), the test order processing apparatus comprising:
   a first display image output part that displays mutually linked one or more test orders and one or more test apparatuses on a display device in a displaying form showing which test order is linked to which test apparatus,
   a test apparatus determination part that determines the test apparatus that should perform the test and one or more test orders linked thereto from the test orders and the test apparatuses linked thereto, which are displayed on the display device, by a user's input or by an automatic selection;
   a test result receiving part that receives each test result sent from the test apparatus(es) connected to the test order processing apparatus; and
   a (test result/test order) linking part that links one or more test results to the one or more test orders linked to the test apparatuses, according to the input sequence or the automatical selection sequence, the test results being received by the test result receiving part from the test apparatus that should perform the test, and the test apparatuses being determined by the test apparatus determination part.
[2] The test order processing apparatus of [1], further comprising:
   a test order receiving part that receives the test order containing one or more test items from a sender of the test order;
   a linking information-holding part that holds information linking said one or more test items contained in the test order to a test apparatus corresponding thereto; and
   a (test order/test apparatus) linking part that refers to the linking information and test item(s) in the test order and links the test order to one or more corresponding test apparatuses.
[3] The test order processing apparatus of [1] or [2], further comprising a second display image output part that displays the apparatus that should perform the test and one or more test orders linked thereto, which are determined by the test apparatus determination part, on the display device in a displaying form showing that they are determined.
[4] The test order processing apparatus of any of [1] to [3], further comprising a test report sending part that sends the test result, linked to the test order by the (test result/test order) linking part, to a given recipient as a test report or a part of a test report.
[5] The test order processing apparatus of [3], wherein
   the first display image output part displays the identifying information of all test apparatuses connected to the test order processing apparatus on the display device, and displays the identifying information of one or more test orders linked to the test apparatus on the display device for each identifying information of each test apparatus, and
   the second display image output part displays the identifying information of the test apparatus that should perform the test and the identifying information of one or more test orders linked thereto determined by the test apparatus determination part from the identifying information of all test apparatuses and the identifying information of one or more test orders linked thereto, which are displayed by the first display image output part, in a different displaying form.
[6] A computer program causing a computer to execute a test order processing to link each test result, sent from a test apparatus to the computer, to a corresponding test order, the computer program making the computer functions as:
   a first display image output part that displays mutually linked one or more test orders and one or more test apparatuses on a display device in a displaying form showing which test order is linked to which test apparatus,
   a test apparatus determination part that determines the test apparatus that should perform the test and one or more test orders linked thereto from the test orders and the test apparatuses linked thereto, which are displayed on the display device, by a user's input or by an automatic selection;
   a test result receiving part that receives each test result sent from the test apparatus(es) connected to the test order processing apparatus; and
   a (test result/test order) linking part that links one or more test results to the one or more test orders linked to the test apparatuses, according to the input sequence or the automatical selection sequence, the test results being received by the test result receiving part from the test apparatus that should perform the test, and the test apparatuses being determined by the test apparatus determination part.
[7] The computer program of [6], further comprising a program making the computer functions as:
   a test order receiving part that receives the test order containing one or more test items from a sender of the test order;
   a linking information-holding part that holds information linking said one or more test items contained in the test order to a test apparatus corresponding thereto; and
   a (test order/test apparatus) linking part that refers to the linking information and test item(s) in the test order and links the test order to one or more corresponding test apparatuses.
[8] The computer program of [6] or [7], further comprising a program making the computer functions as a second display image output part that displays the test apparatus that should perform the test and one or more test orders linked thereto, which are determined by the test apparatus determination part, on the display device in a displaying form showing that they are determined.
[9] The computer program of any of [6] to [8], further comprising a program making the computer function as a test report sending part that sends the test result, linked to the test order by the (test result/test order) linking part, to a given recipient as a test report or a part of a test report.

Using the test order processing apparatus or computer program of the present invention, even when a medical institution has a test order ID send/receive non-compliant test apparatus, the test result data output from such test apparatus can be linked to the truly corresponding test order that requested to perform the test by simply selecting the test apparatus prior to the test by the test apparatus. As a result, the linking work (input of test order ID and the like) between the test results and the test order ID becomes unnecessary, which in turn prevents a mistake in inputting character strings of the test order ID and prevents linking mistakes. In addition, the problem of time loss is resolved which is found in conventional test order processing apparatuses and prevents the linking work from being performed until the test results are received from the test order ID send/receive non-compliant test apparatus.

### BRIEF DESCRIPTION OF THE DRAWINGS

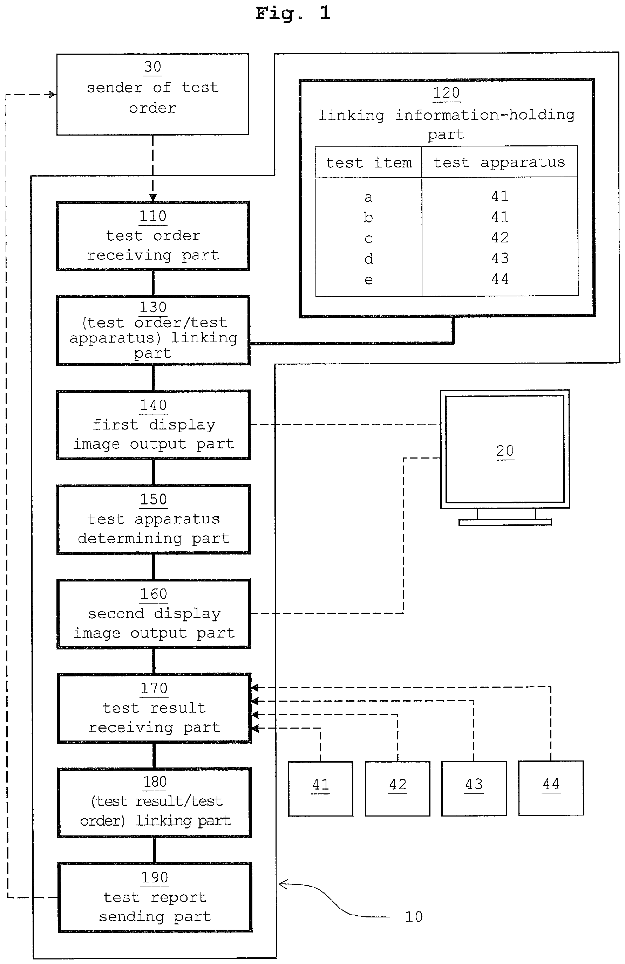

Fig. 1 is a block diagram showing a preferred embodiment of the constituent of the test order processing apparatus of the present invention, which is also a block diagram showing one embodiment of the constituent of a test system using the test order processing apparatus. The communication between the test order processing apparatus of the present invention and outside apparatuses (display device, sender of test order, test report recipient, test apparatus) is shown with a broken line. In the embodiment of Fig. 1, the sender of the test order is also the recipient of the test report.

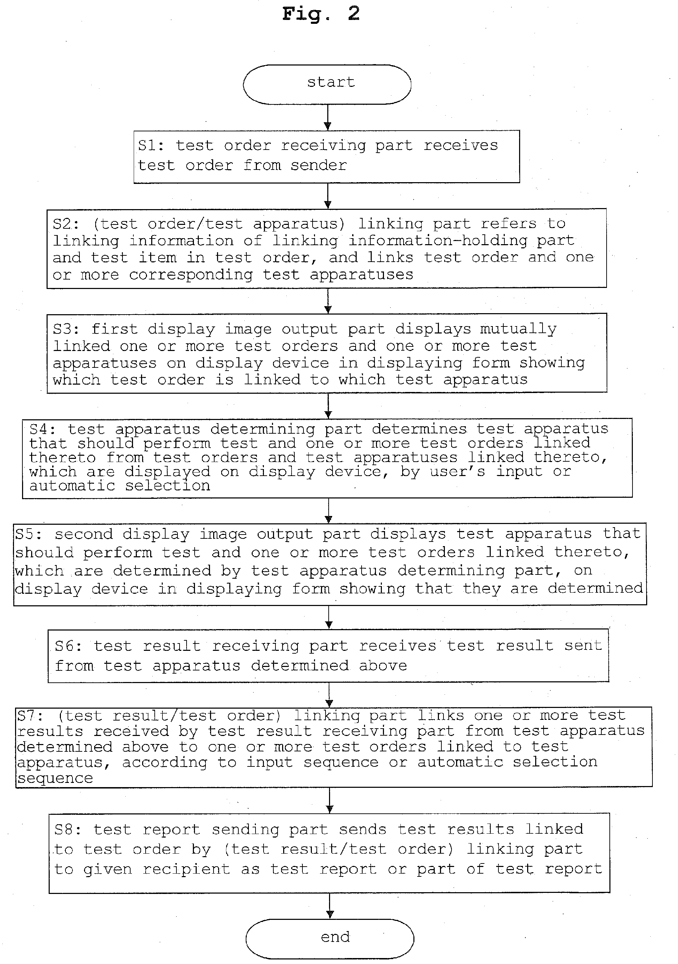

Fig. 2 is a flowchart showing the flow of the processing of the test order performed in the preferred embodiment of the test order processing apparatus in the present invention, The flowchart is also a flowchart showing the flow of the processing of the test order in the program of the present invention.

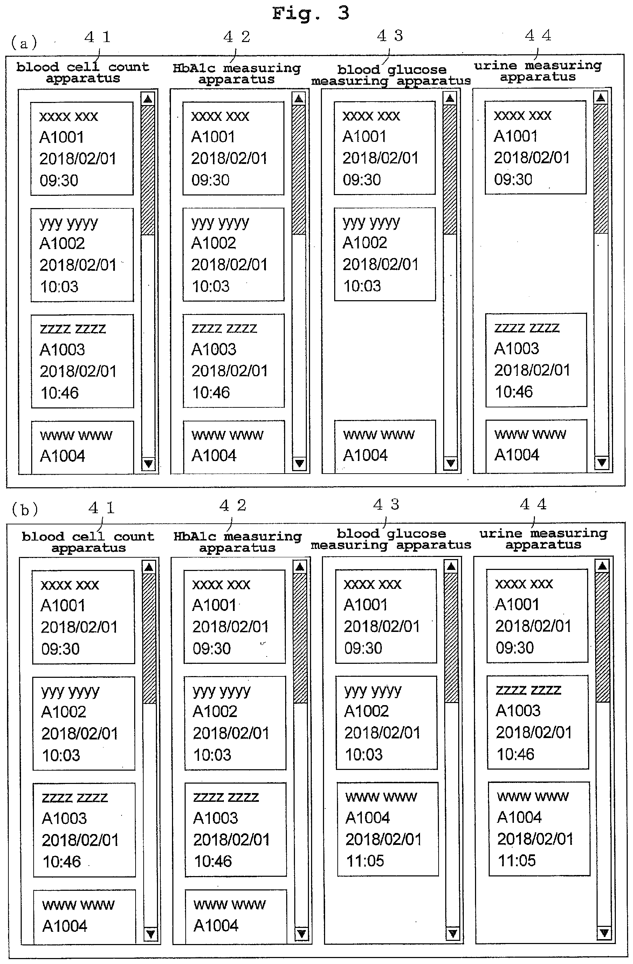

Fig. 3 shows, in the preferred embodiment of the present invention, one embodiment of a display image (display screen) displayed on a display device by the first display image output part.

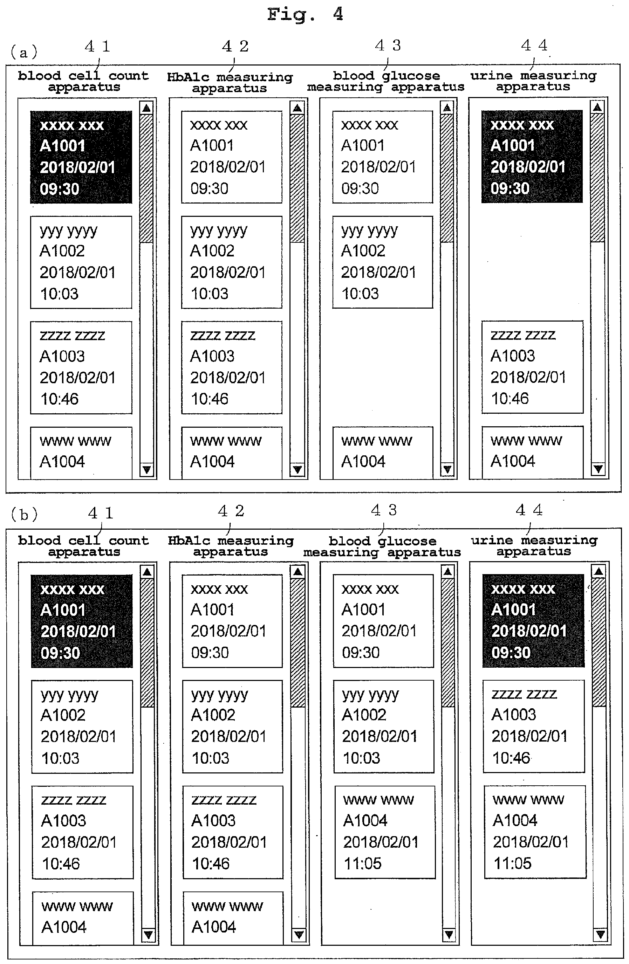

Fig. 4 shows, in the preferred embodiment of the present invention, one embodiment of a display image (display screen) displayed on a display device by the second display image output part. The display embodiment of Fig. 4(a) corresponds to the display embodiment of Fig. 3(a), and the display embodiment of Fig. 4(b) corresponds to the display embodiment of Fig. 3(b).
Fig. 5 is a block diagram showing an example of conventional connection state of a doctor-side computer, a test order processing apparatus (computer) provided in the test department, and various test apparatuses provided in the test department. In this Figure, display devices in the doctor-side computer and the test order processing apparatus provided in the test department show the matters (test order ID, test subject ID, test item) contained in the test order, and suggest that these matters are sent and received as data between the computers. The dashed line shows the communication pathway between the computers (including computer as a control part of test apparatus).

The symbols in the Figures mean the following: 10; test order processing apparatus, 20; display device, 30; sender of test order, 41 - 44; test apparatus, 110; test order receiving part, 120; linking information-holding part, 130; (test order/test apparatus) linking part, 140; first display image output part, 150; test apparatus determination part, 160; second display image output part, 170; test result receiving part, 180; (test result/test order) linking part, 190; test report sending part.

### DETAILED DESCRIPTION OF THE INVENTION

The present invention is explained in detail in the following by referring to Examples.

Each part constituting the test order processing apparatus of the present invention (hereinafter to be also referred to as the processing apparatus) may each be constructed using a combination of an electron circuit, an electric circuit and an independent processing apparatus. In a preferred embodiment, each of these parts is constituted using a computer and a program to be executed by the computer.

In the following, a preferred embodiment and an effect of each part are explained by illustrating an embodiment in which the processing apparatus is constituted using a computer. The constituent of each part shown below can be performed partially or entirely by a combination of an electronic circuit, an electric circuit, an independent processing apparatus and the like.

Fig. 1 is a block diagram showing an embodiment of the constituent of the processing apparatus or program of the present invention, which is also a block diagram showing one embodiment of the constituent of a test system using the processing apparatus. As shown in Fig. 1, the processing apparatus 10 is an apparatus that mediates the computer 30 of the sender (doctor-side) that transmits the test order and the test apparatus (41 - 44), appropriately links and displays the test order and the test apparatus and, even when the test apparatus is test order ID send/receive non-compliant, appropriately links the test result data (test data) output from such test apparatus to the truly corresponding test order.

In the example of Fig. 1, for the sake of explanation, the test apparatuses 41, 42, 43, 44 are connected to the processing apparatus 10, and test apparatus 44 is test order ID send/receive non-compliant. The other test apparatuses 41 - 43 can send and receive test order ID and the like from the test order processing apparatus. In this example, only the test results sent from the test apparatuses 41 - 43 are used, similar to the test apparatus 44.

The test order ID send/receive non-compliant test apparatus 44 does not receive the data of the test order, test order ID and the like from the test order processing apparatus 10. However, the test order processing apparatus can utilize the function of outputting the test results through a particular interface to an outside computer. The test apparatus 44 is connected to the test order processing apparatus 10 via the interface, and the processing apparatus 10 can receive test results from the test apparatus 44.

As exemplified in Fig. 1, the processing apparatus at least has a (test order/test apparatus) linking part 130, a first display image output part 140, a test apparatus determination part 150, a test result receiving part 170, and a (test result/test order) linking part 180. In the example of Fig. 1, as a preferred embodiment, a test order receiving part 110, a linking information-holding part 120, a second display image output part 160 and a test report sending part 190 are further provided.

### (test order receiving part)

The test order receiving part 110 receives the test order containing one or more test items from a sender (computer 30) of the test order, and the test order receiving part 110 can receive the test order one after another. Similar to the description in the BACKGROUND OF THE INVENTION, a test order contains at least a test item necessary for diagnosis and a test order ID as the identifying information of the test order itself. In this example, as a simple example for explanation, each test order contains test order ID, full name as identifying information (test subject ID) of the test subject, and a test item requiring a specimen test, and the test items contained in the test order are five items (a, b, c, d, e) at maximum and each test order contains any test item of these test items. The test order ID may be the same as the test subject ID. The information contained in the test order may be the same as the information contained in a conventionally-known test order.

### (linking information-holding part)

The linking information-holding part 120 holds information linking said one or more test items contained in the test order to test apparatuses corresponding thereto. In the example of Fig. 1, the substance of the linking information-holding part is a storage device, and the storage device stores linking information as database (hereinafter to be also referred to as linking database) that links various test items a, b, c, d, e to be contained in the test order to respective test apparatuses 41, 42, 43, 44 corresponding to the test items, and functions as a linking information-holding part. In this example, test items a, b are linked in advance to the test apparatus 41, test item c is linked to the test apparatus 42, test item d is linked to the test apparatus 43, and test item e is linked to the test apparatus 44. The linking information-holding part may be formed in the program.

The linking database stores in advance all test items contained in a test order and all test apparatuses corresponding to any of the test items and communicatably connected to the processing apparatus, as the linking information in which they are linked in advance and in a form a computer can refer to. While the structure of the linking database is not particularly limited, a form permitting quick and easy reference by the (test order/test apparatus) linking part such as table and the like is preferable.

### ((test order/test apparatus) linking part)

The (test order/test apparatus) linking part 130 refers to the linking information and test item in the test order, and links the test order to the corresponding one or more test apparatuses. For example, when test items contained in a test order are a, b, c, e, the (test order/test apparatus) linking part 130 links test items a, b to the test apparatus 41, test item c to the test apparatus 42, and test item e to the test apparatus 44. The (test order/test apparatus) linking part 130 links test orders sent one after another to the corresponding one or more test apparatuses and can store them one after another.

To link a test order to a test apparatus means, for example, to link identifying information of a test order to identifying information of a test apparatus.

### (the first display image output part)

The first display image output part 140 displays mutually linked one or more test orders and one or more test apparatuses on a display device 20 in a displaying form showing which test order is linked to which test apparatus so that the user can identify that they are linked to each other. The display device may be composed of plural display devices, and the screen of the display device may display plural screens and plural windows. In a preferred embodiment, the first display image output part 140 displays on the display device 20 one or more test orders and one or more test apparatuses linked to each other by the (test order/test apparatus) linking part 130.

The user is a person who operates the processing apparatus and is typically a test staff. It may also be a dedicated operator or the like who operates the processing apparatus.

The displaying form showing which test order is linked to which test apparatus means, for example, a display of test identifying information of the apparatus linked to the identifying information of the test order, more specifically, any form of display that allows the user to identify that the two are related to each other, such as a state in which the identification information of the test order is displayed in the column of identification information of the test apparatus, a state in which the identification information of the test apparatus is displayed in the column of the identification information of the test order, a state in which the identification information of the test order and the identification information of the test apparatus are arranged close to each other, and the like. When only one test apparatus is connected to the processing apparatus, all test orders are linked only to the single test apparatus, and the test is performed only by the test apparatus. In such a case, as a displaying form showing which test order is linked to which test apparatus, only the received test order may be displayed without displaying the test apparatus. Such form of displaying only the test order is included in the "displaying form showing which test order is linked to which test apparatus" in the present invention since the user can identify linking to only one test apparatus.

The first display image output part 140 may cause the display device 20 to successively display the test order and one or more test apparatuses which are successively linked by the (test order/test apparatus) linking part 130. In a preferred embodiment, the (test order/ test apparatus) linking part 130 arranges and displays in chronological order so that the sequence of receipt of the test orders by the user is known. A preferred embodiment of the display mode is described later.

### (test apparatus determination part)

The test apparatus determination part 150 determines the test apparatus that should perform the test and one or more test orders linked thereto from the aforementioned one or more test apparatuses linked to the test order, which are displayed on the display device 20, by the user's input or automatic selection by the processing apparatus. This example is an embodiment in which the user inputs. The automatic selection by the processing apparatus is described below as a deformation embodiment. In this example, for the aforementioned input by the user, the processing apparatus has an input apparatus (not shown), and the user can select and input the test apparatus that should perform the test and a test order linked thereto. In addition, the processing apparatus or the aforementioned test apparatus determination part have an input receiving part (not shown) that receives an input by the user from the input device. The test apparatus determination part 150 receives the input as a selection result by the user and determines the test apparatus that should perform the test and a test order linked thereto.

In this example, one test apparatus that should perform the test and one test order linked thereto are selected and input by the user, and they are linked to each other. On the other hand, in the deformation embodiment of the present invention, one test apparatus that should perform the test and one or more any number of test orders linked thereto may be selected and input. In an embodiment in which plural test orders are selected, the sequence of input is the information used for linking the test result obtained later and the test order. Such deformation embodiment is mentioned below.

### (second display image output part)

The second display image output part 160 displays, on the display device, the (test apparatus that should perform the test and test order linked thereto) determined by the test apparatus determination part 150 in a displaying form indicating that they are determined (i.e., user who saw the display can identify that the selection of the user has been accepted). For example, as mentioned above, while watching the display by the first display image output part 140, the user selects and inputs the test apparatus that should perform the test and one test order linked thereto. The second display image output part 160 displays the selected and input results (test apparatus that should perform the test and one test order linked thereto) in a displaying form showing that the user selected (i.e., to distinguish other test apparatus from test order linked thereto). The displaying form indicating that the user has selected may be any form as long as it can be distinguished from others and is not particularly limited. For example, various displaying forms that can visually distinguish the decision of the user from other displays can be used, such as change of the background color or character color of the display, inversion of the background color and character color, enclosing with a frame, addition of underline or symbol, emphasis with bold lettering, display on another screen and the like.

### (test result receiving part)

The test result receiving part 170 can receive respective test results any time that are sent from the test apparatuses (41, 42, 43, 44) connected to the processing apparatus 10.

The processing apparatus or test result receiving part 170 is preferably provided with a (test result/test apparatus) linking part (not shown) that links the test results sent from the test apparatus to the corresponding test apparatus. By referring to the linking information between test results and test apparatus by the (test result/test apparatus) linking part, the received test results are correctly linked to the truly corresponding test order through a test apparatus linked thereto.

### ((test result/test order) linking part)

The (test result/test order) linking part 180 is an important part that enables, together with the test apparatus determination part 150, linking of the test results (test results without accompanying identifying information of test order) received from the test order ID send/receive non-compliant test apparatus to the truly corresponding test order that requested the test.

In this example, the (test result/test order) linking part 180 links, after one test apparatus that should perform the test (e.g., test apparatus 44 linked to test order ID (A1001)) is determined by the test apparatus determination part 150, the test results received by the test result receiving part 170 from the test apparatus 44 as the first test result received after the determination to the test order (test order ID (A1001)) linked to the test apparatus 44. At this time, it is also possible to refer to the linking information by the aforementioned (test result/test apparatus) linking part (not shown). As mentioned above, the case where plural test orders are selected for one test apparatus is described later as a deformation embodiment.

By the linking by the (test result/test order) linking part, the test results without accompanying identifying information of test order are easily and correctly linked to the truly corresponding test order ID (A1001) without a linking operation by the user.

### (test report sending part)

In a preferred embodiment of the processing apparatus, as shown in Fig. 1, a test report sending part 190 is further provided. The test report sending part 190 sends back the test results correctly linked to the test order by the (test result/test order) linking part 180 to a given recipient (e.g., sender 30 of test order and the like). The test results linked to the test order may be sent singly (without integrating into one test report) to the recipient as a part of the test report to the test order requesting plural tests, or plural test results linked to the same test order may be integrated and sent to the recipient as a test report satisfying all requests of the test order. When a test item contained in a test order corresponds to only one test apparatus, the test results from the one test apparatus become a test report satisfying the requests of the test order.

### (test report formation part)

A test report formation part that integrates plural test results linked to the same test order and forms a test report may be further provided in the preceding stage of the test report sending part 190. As a result, plural test results are automatically integrated into one test report, and a burden of operation on the user during reporting the test results are drastically improved. When the test results are sent singly, a test report formation part that integrates plural test results linked to the same test order and forms a test report may be provided in the recipient computer.

Specific preferred operation of the above-mentioned processing apparatus and user's operation are described with reference to the flowchart of Fig. 2. Among the following steps S1 to S7, steps S3 to S6 are important as the main steps. Further, the flowchart shows a case where the user selects and inputs (test apparatus that performs measurement and test order linked thereto). An embodiment in which the (test apparatus that performs measurement and test order linked thereto) are automatically selected is described below.

First, a doctor or the like operates a computer (e.g., a computer or the like on which an electronic medical records are executed) as the sender of the test order, and sends a test order. As described above, the data of the test order includes test order ID, full name as test subject ID, and test items requiring a sample test. The test subject ID may be an identification number or the like accorded to the test subject.

In step S1, a test order receiving part 110 of the processing apparatus receives a test order from a sender.

In step S2, a (test order/test apparatus) linking part 130 refers to the linking information of the linking information-holding part 120 and the test item in the test order, links the test order and one or more compatible test apparatuses, and stores these in a storage device.

In step S3, the first display image output part 140 displays the aforementioned test order and one or more test apparatuses linked by the (test order/test apparatus) linking part on the display device 20 so that the user can identify that they are linked to each other.

In step S4, the user first looks at the screen of the display device 20 and confirms the presence of the test order. The user looks at the screen, selects a test apparatus (e.g., test apparatus 44) the user intends to use for the measurement and the test order linked thereto (e.g., test order ID; A1001), and inputs them into the processing apparatus. The selection of the user may be that of the test order (A1001) linked to the test apparatus 44 or the test apparatus 44 linked to the test order (A1001). When many test orders are linked to one test apparatus, one or more test orders may be selected for one test apparatus. When plural test orders are selected for one test apparatus, selection (input) sequence is stored. This is described below. Next, the test apparatus determining part 150 determines a test apparatus that should perform the test 44 and one test order (A1001) linked thereto by the aforementioned user's input. An embodiment of automatic selection is described below.

In step S5, the second display image output part 160 displays the test apparatus that should perform the test and the test order linked thereto determined by the test apparatus determining part 150 on a display device 20 in a displaying form showing that they are determined. As a result, the user correctly knows that the test apparatus that should perform the test and the test order have been selected.

In step S6, the user (excluding operators not eligible for testing) sets a specimen and the like in the selected test apparatus (e.g., test apparatus 44) and performs the test. On completion of the test, the test apparatus 44 sends the test result data to the processing apparatus. The test result receiving part 170 of the processing apparatus receives the test results from the test apparatus 44.

In step S7, the (test result/test order) linking part 180 links, after test apparatus 44 and the test order linked thereto are determined by the test apparatus determining part 150, the test results received by the test result receiving part 170 from the test apparatus 44 as the first test results to the test order (A1001) linked to the test apparatus 44. An embodiment in which plural test orders are selected for one test apparatus is described below. The (test result/test order) linking part 180 confirms the presence of a test order (A1001) to be linked to the received test results and when it is present, performs the aforementioned linking. When a test order (A1001) to be linked is not present for some reason, the test results may be stored as, for example, an object waiting for linking processing. The presence of an object waiting for linking processing may be displayed to the user.

Step S8 is a step executed in a preferred embodiment, in which the (test result/test order) linking part 180 sends the test results linked to one test order to a given recipient as a test report or as a part of a test report.

A preferred embodiment of the processing apparatus is described in the following.

In the preferred embodiment of the present invention, the first display image output part 140 displays the identifying information of all the test apparatuses 41 - 44 connected to the processing apparatus on the display device and, for the identifying information of each test apparatus, displays the identifying information of one or more test orders linked to the test apparatus on the display device. Then, the second display image output part 160 displays the identifying information of the test apparatus that should perform the test and the identifying information of the test order linked thereto, which are determined by the test apparatus determining part 150, from the identifying information of all test apparatuses and the identifying information of one or more test orders linked thereto, which are displayed by the first display image output part 140, in a different displaying form identifiable by the user. Fig. 3 and Fig. 4 show specific display examples of this embodiment.

Fig. 3 shows a preferred embodiment of a display image (display screen) displayed on the display device 20 by the first display image output part 140. Fig. 4 shows a preferred embodiment of a display image displayed on the display device by the second display image output part 160. The user looks at the display image of Fig. 3, selects and inputs the test apparatus and the test order, based on which the test apparatus determining part 150 determines the test apparatus that should perform the test and the test order and the second display image output part 160 displays the display image of Fig. 4.

In the examples of Fig. 3 and Fig. 4, the columns of 4 test apparatuses are respectively arranged in one display screen. Identification information (product number) of each test apparatus is displayed at the top of the column of each test apparatus. The identifying information of each test apparatus is "blood cell count apparatus" for test apparatus 41, "HbA1c measuring apparatus" (hemoglobin A1c measuring apparatus) for test apparatus 42, "blood glucose measuring apparatus" for test apparatus 43, and "urine measuring apparatus" for test apparatus 44. In the examples of Fig. 3 and Fig. 4, among these test apparatuses 41 to 44, the test apparatuses 42, 44 are test order ID send/receive non-compliant. The identifying information of the test apparatus may be name, initial, icon, abbreviation entered by the user or the like.

In the columns of test apparatuses 41 to 44, items showing the test order (full name as test subject ID, test order ID, creation date of test order) are displayed. The creation date of the test order may also be the date and time when the test order was sent or the like. In a preferred embodiment, these displays were received by the test order receiving part 110 and linked by the (test order/test apparatus) linking part 130.

In the examples of Fig. 3 and Fig. 4, a test order displayed on the upper side of each column is a test order having an older reception date and time. A scroll bar is provided individually in each column and the scroll bar makes it possible to display lower test orders not displayed in the column by raising them up to the inside of the column. With such a display mode, the user can always pay attention to the oldest examination order displayed at the top of each column, and can preferably use each column as a display device of each test apparatus.

In the example of Fig. 3(a), all 4 test apparatuses 41 to 44 display the test order (test order ID: A1001). This means that the test order (A1001) contains all test items a, b, c, d, e. In contrast, the test order (A1002) and test order (A1003) are displayed in only a part of 4 test apparatuses 41 to 44. This means that the test order (A1002) does not contain test item e and that the test order (A1003) does not contain test item d. The same applies to the example of Fig. 3(b).

Fig. 3(a) and Fig. 3(b) have different display embodiments. In the example of Fig. 3(a), the column of a test apparatus not linked to the test order does not display the test order and is empty (e.g., test order (A1002) in the column of the test apparatus 44, test order (A1003) in the column test apparatus 43). Such display mode is advantageous in that the same test order is arranged in a row, and the constitution of the test items of the test order and unprocessed test are easy to understand. On the other hand, in the example of Fig. 3(b), the column of each test apparatus does not contain an empty column as in Fig. 3(a), and the test orders are sequentially displayed simply from older ones. Such display mode is advantageous in that attention can be always paid to the test order at the top of each column since all the test orders requiring testing are located within the column.

In the example of Fig. 3, the columns of 4 test apparatuses 41 to 44 are present in the region of one screen. When a column of further test apparatus is added, an embodiment is available in which all columns are displayed in one screen by the horizontal scroll bar. In addition, since a test apparatus compliant with sending and receiving of test order ID does not require linking by the present invention, the processing apparatus may be constituted to hide the column of the test apparatus compliant with sending and receiving of test order ID.

In the example of Fig. 4, a test order (A1001) of test apparatus 41 and a test order (A1001) of test apparatus 44 are selected and input by the user, and displayed with reverse colors of the characters and background so that the user can recognize them. When a plurality of test orders are selected for one test apparatus, the sequence of selection is held as described later so as to make the linking by the (test result/test order) linking part only one way. It may be possible to select multiple test apparatuses for one test order. Since the test time for each test apparatus is different from each other, the test orders located at the top of each test apparatus may be different from each other. A plurality of different combinations of test orders and test apparatuses may be selected (up to four in the example of Fig. 4).

In the example of Fig. 4, the selection result of the user is input by positioning the pointer on the object test order, and then clicking. In a preferred embodiment, a test order displayed by clicking once (e.g., inverted displaying state) may return to a state free of selection (the original display state) by clicking again. When the pointer is moved on the test order, the region of the test order can be changed (e.g., emphasized expression such as changing to a light color, surrounding with a frame, adding an underline, bold lettering and the like) to be distinguishable, whereby which test order the user is about to select is clearly displayed.

Any input device and input method can be adopted for input of user selection results. For example, a mode in which a touch panel is adopted and the object test order is pressed to input, a mode in which the cursor is moved to an object test order by the up, down, left, and right direction keys and input by pressing the input key and the like can be mentioned.

In a preferred embodiment, a test order displayed in the selected state may be deleted from the display screen at the time when linked to the test result, or when the test report sending part determines that the recipient has received the test result.

When a modified test order such as addition or deletion of measurement items is received, the test order of each test apparatus may be added or deleted accordingly. For example, in the examples of Fig. 3, Fig. 4, when a test order (A1002) containing test items a, b, c, d is received, the test order (A1002) is changed and a test order (A1002) without measurement item c is newly received thereafter, corresponding test order (A1002) is deleted from the column of the test apparatus 43.

These displays may be performed by the second display image output part or a further different display image output part.

### (embodiment in which plural test orders are selected for each test apparatus in test apparatus determining part)

In a preferred embodiment of the test apparatus determining part, plural test orders may be selected for each test apparatus. When plural test orders are selected for each test apparatus, the sequence of entry by the user, or the sequence of automatic selection in the case of automatic selection, is stored in, for example, selection sequence storage part (not shown) so that the test results sent from the selected test apparatus can be correctly linked to the corresponding test order (test order to be linked to test result) from the aforementioned plural test orders. The selection sequence storage part may be formed in a storage device or program. When, for example, for one test apparatus 44 in Fig. 1, when plural test orders linked thereto are selected (by user's input or automatic selection), the sequence of selection is stored in a selection sequence storage part (not shown). Thereafter, when plural test results are sequentially sent from the test apparatus 44, the (test result/test order) linking part refers to the sequence of selection stored in the selection sequence storage part, the test result and the test order are linked to each other according to the sequence of receptance and the sequence of selection corresponding thereto. For example, the test result sent firstly is linked to the test order selected firstly, and the test result sent secondly is linked to the test order selected secondly.

As a result, even if a plurality of test orders linked to each test apparatus are selected, a plurality of test results from each test apparatus can be correctly linked to the corresponding test order.

### (embodiment in which test order is automatically selected in test apparatus determining part)

In a preferred embodiment of the test apparatus determining part, a test order automatically selecting part may be further provided in which a test order is automatically selected in place of the user's input. In this embodiment, the user merely make the test apparatus perform the test, according to the test apparatus and test order automatically selected and displayed by the processing apparatus. When the test order automatically selecting part selects plural test orders for one test apparatus, for example, the test order automatically selecting part is constituted to select a test order from the oldest, and the sequence of selection is stored in the above-mentioned selection sequence storage part. The user makes the test apparatus perform the tests of automatically selected and displayed test orders from the oldest. As a result, even when plural test orders are selected for each of plural test apparatuses, if the test is performed in the exact sequence of selection, they can be correctly linked to the original test orders. The test order automatically selecting part may be constituted to receive instructions to select other computer (e.g., host computer and the like that uses the processing apparatus as terminal) connected to the processing apparatus.

Whether selection (determination) of the test apparatus and the test order linked thereto is performed by the user's manual input, or automatically performed by the test order selecting part may be selected by the user by switching the operation mode. In either case of selection by user's input and automatic selection by the test order selecting part, the test apparatus determining part determines the test apparatus that should perform the test and the test order linked thereto based on the selection results. The second display image output part displays the determined test apparatus that should perform the test and the test order linked thereto on the aforementioned display device as mentioned above.

According to the embodiment, the processing apparatus preferably contains a selection results receiving part. The select results receiving part is constituted to receive an input selected by the user, and automatically generated selection results by the aforementioned test order automatic select. The test apparatus determining part refers to the selection results received by the select results receiving part and can determine a test apparatus that should perform the test and the test order linked thereto, irrespective of whether the selection of the test apparatus and the test order linked thereto is made manually, automatically, semi-automatically or the like.

### (computer program of the present invention)

The computer program of the present invention (the program) is used for constituting each part of the above-mentioned test order processing apparatus of the present invention by using a computer. The program is a computer program constituted to make the computer function at least as the above-mentioned first display image output part 140, the above-mentioned test apparatus determining part 150, the above-mentioned test result receiving part 170, and the above-mentioned (test result/test order) linking part 180. The above-mentioned linking information-holding part 120 may be separately configured using a storage device or may be configured in the program. In a preferred embodiment, the aforementioned program may contain a computer program constituted to make the computer function as the above-mentioned test order receiving part 110, the above-mentioned (test order/test apparatus) linking part 130, the above-mentioned second display image output part 160. In addition, the program may contain a computer program constituted to make the computer function as test report sending part 190, and further, the detailed action of test report formation parts 110 to 190 are described above. The program can be appropriately modified such that it functions as in the above-mentioned preferred embodiment. The program may be stored in a computer-readable recording medium and supplied to the users or supplied to the computers of users via internet and the like.

### (sender)

The sender 30 of the test order shown in Fig. 1 is not particularly limited and may be an apparatus (particularly, computer) capable of communicating with the processing apparatus. It may be a sender according to the network of the system using the processing apparatus such as a terminal computer used by a doctor or the like for creation of electronic medical records and the like (from which computer a test order is issued), a computer of a management department that receives a test order from the terminal computer and sends the test order to the test order processing apparatus or the like.

### (recipient)

On the other hand, the given recipient to which the test report or test result is sent is not particularly limited and may be any as long as it is a computer capable of communicating with the processing apparatus. It may be a recipient predetermined according to the network of the system using the processing apparatus or flow of a test report such as a terminal computer used by a doctor or the like, a computer of a management department that receives a test report from the processing apparatus and sends the test report to a terminal computer of a doctor etc., or the like. In the network system of a general medical institution, the sender (issuing source) of a test order is often a recipient of the test report (e.g., when a test order is sent from a doctor's terminal computer (issuing source) and the test report is sent back to the doctor's terminal computer and the like). However, the sender of the test order and the recipient of the test report for the processing apparatus may be different from each other.

The sender and the recipient may also be contained in the test order processing apparatus itself. For example, the processing apparatus may also be a terminal computer used by a doctor and the like, and electronic medical record program and the like. A program that issues a test order and the program of the present invention may be executed by the same computer. In this case, the program that issues a test order is constituted to transfer the data of the test order to the test order receiving part 110 of the program of the present invention, and the test report sending part 190 may be constituted to transmit test results to the program that gives the test order.

### (embodiment of processing apparatus as computer)

The processing apparatus can appropriately have input devices such as keyboard, mouse, touch panel, and the like.

The processing apparatus is an apparatus that imparts operability as if a display screen provided for a test apparatus capable of sending and receiving test order ID is given to a test order ID send/receive non-compliant test apparatus. Furthermore, as shown in the below-mentioned Fig. 3 and Fig. 4, it is an apparatus that imparts operability as if each display screen of all test apparatuses connected to the processing apparatus are collectively displayed on one screen of one display apparatus and simultaneously displayed.

As described along the flowchart of Fig. 2, the user performs the selected operation while looking at the display screen of the display device, sets specimen and the like on the selected test apparatus, and starts the test. From such aspect, the processing apparatus is preferably a tablet-type computer that can be carried to the vicinity of test apparatus or in an embodiment in which the display screen and the input device (preferably a touch panel) are separated from the main part of the computer by a wireless connection. This enables the user to approach the test apparatus together with the processing apparatus and preferably operate the processing apparatus as if it is a part of the operation panel of the test apparatus.

### (test subject)

The test subject may be not only a human but also an animal other than human. When the test subject is a human, the test subject may include not only patients having a disease but also healthy subjects not having a disease. For example, in a medical examination and the like, any medical examinee is the subject irrespective of the presence or absence of a disease and the test data of given test items are obtained.

### (test item contained in test order)

The test items are not particularly limited and may be those contained in various test orders such as test items relating to specimen testing using specimens (blood, urine, feces, cell and the like) collected from the test subject as the test targets, test items using the body of the test subject as the test target such as electrocardiographic measurement, ultrasonic diagnosis, radiographic test, CT scan and the like, and the like. The usefulness of the present invention becomes particularly noticeable when the test items relate to specimen testing.

Examples of the test items relating to specimen testing include test items about hematological test, biochemical test, immunological test, genetic test, and the like. The specific test items recited below are not necessarily classified into only one of the above-mentioned tests and may be classified into, for example, both hematological test and biochemical test and the like.

Examples of test items relating to blood count include white blood cell count, erythrocyte count, hemoglobin concentration, hematocrit value, average erythrocyte volume, mean corpuscular hemoglobin, mean corpuscular hemoglobin concentration, platelet count, erythrocyte distribution width, mean platelet volume, platelet distribution width, platelet crit, lymphocyte count, lymphocyte ratio, monocyte count, monocyte ratio, granulocyte count, granulocyte ratio, neutrophil count, neutrophil ratio, eosinophil count, eosinophil ratio, basophil count, basophil ratio, atypical lymphocyte count, atypical lymphocyte ratio, large juvenile cell count, large juvenile cell ratio, reactive protein concentration, blood glucose level, ammonia, sodium, potassium, chlorine and the like.

Examples of test items relating to hemoglobin A1c (HbA1c) include glycohemoglobin A1c, hemoglobin F (HbF), urine albumin, urine creatinine, urine AC ratio, high sensitivity C-reactive protein (HSCRP) concentration, cystatin and the like.

Examples of test items targeting urine include color, turbidity, glucose, protein, bilirubin, urobilinogen, pH, specific gravity, occult blood, ketone body, nitrite salt, leukocyte test, ascorbic acid, creatinine, µ albumin, p/c ratio (protein-creatinine ratio), a/c ratio (albumin-creatinine ratio), urea nitrogen, uric acid, total cholesterol, ammonia, neutral fat, total protein, albumin, total bilirubin, calcium, inorganic phosphorus, direct bilirubin, HDL cholesterol, magnesium, γ-glutamyl transpeptidase, AST (GOT), ALT (GPT), creatine kinase, lactic acid dehydrogenase, alkaline phosphatase, leucine aminopeptidase, CK-MB, choline esterase, amylase, lipase, sodium, potassium, chloride and the like.

Examples of biochemical test items include total protein, albumin, neutral fat, total cholesterol, LDL cholesterol, HDL cholesterol, AST (GOT), ALT (GPT), γ-glutamyl transpeptidase, alkaline phosphatase, lactic acid dehydrogenase, choline esterase, total bilirubin, direct bilirubin, leucine aminopeptidase, ammonia, amylase, lipase, urea nitrogen, creatinine, uric acid, glucose, creatine kinase, calcium, inorganic phosphorus, magnesium, hemoglobin A1c, sodium, potassium, chloride, ketone body, SAA, C-reactive protein (CRP), hemoglobin concentration, CK-MB, cCRP, fructosamine, rheumatoid factor quantification and the like.

Examples of genetic test items include gene test, chromosome test, DNA test, RNA test and the like.

### (test apparatus)

As the test apparatus, an apparatus communicatably connected to the processing apparatus such that at least test result can be sent and capable of measurement and analysis relating to the above-mentioned test items can be used. Examples of test apparatus relating to specimen testing include blood cell counting apparatus, blood analyzing apparatus, concentration measuring apparatus, immunological measuring apparatus, genetical analyzing apparatus and the like. Only one test apparatus may be able to process only one test item, or one test apparatus may be able to process plural test items.

The connection between the processing apparatus and the test apparatus is not particularly limited, and any connection capable of data communication between a computer and an external apparatus such as connection via wired or wireless LAN, USB, RS-232C, GPIB, blue tooth (registered trade mark), internet, a combination of these and the like can be used.

The number of the test apparatuses that can be connected to the processing apparatus may be one or more. The usefulness of the present invention becomes noticeable when the test order ID send/receive non-compliant test apparatus is contained and the usefulness of the present invention becomes more noticeable when plural test apparatuses are involved.

The processing apparatus may be, as described in the above-mentioned patent document 1, provided with the function of a test order processing apparatus capable of sending and receiving data of test order ID and the like with a test apparatus capable of sending and receiving a test order. The conventional function and the function of the test order processing apparatus of the present invention may be switched.

### INDUSTRIAL APPLICABILITY

According to the present invention, even when the test apparatus contains a test order ID send/receive non-compliant test apparatus, the work of linking the data of the test results sent from such test apparatus to the corresponding test order can be facilitated. The processing apparatus and the program are used not only for diagnosis at medical institutions but also for all fields of use where a test is performed by a test apparatus according to a test order and the test results are sent back.

## Claims

1. A test order processing apparatus for linking a test result sent from a test apparatus to a test order corresponding to the test result), the test order processing apparatus comprising:
a first display image output part that displays mutually linked one or more test orders and one or more test apparatuses on a display device in a displaying form showing which test order is linked to which test apparatus,
a test apparatus determination part that determines the test apparatus that should perform the test and one or more test orders linked thereto from the test orders and the test apparatuses linked thereto, which are displayed on the display device, by a user's input or by an automatic selection;
a test result receiving part that receives each test result sent from the test apparatus(es) connected to the test order processing apparatus; and
a (test result/test order) linking part that links one or more test results to the one or more test orders linked to the test apparatuses, according to the input sequence or the automatical selection sequence, the test results being received by the test result receiving part from the test apparatus that should perform the test, and the test apparatuses being determined by the test apparatus determination part.

2. The test order processing apparatus according to claim 1, further comprising:
a test order receiving part that receives the test order containing one or more test items from a sender of the test order;
a linking information-holding part that holds information linking said one or more test items contained in the test order to a test apparatus corresponding thereto; and
a (test order/test apparatus) linking part that refers to the linking information and test item(s) in the test order and links the test order to one or more corresponding test apparatuses.

3. The test order processing apparatus according to claim 1 or 2, further comprising a second display image output part that displays the apparatus that should perform the test and one or more test orders linked thereto, which are determined by the test apparatus determination part, on the display device in a displaying form showing that they are determined.

4. The test order processing apparatus according to any one of claims 1 to 3, further comprising a test report sending part that sends the test result, linked to the test order by the (test result/test order) linking part, to a given recipient as a test report or a part of a test report.

5. The test order processing apparatus according to claim 3, wherein
the first display image output part displays the identifying information of all test apparatuses connected to the test order processing apparatus on the display device, and displays the identifying information of one or more test orders linked to the test apparatus on the display device for each identifying information of each test apparatus, and
the second display image output part displays the identifying information of the test apparatus that should perform the test and the identifying information of one or more test orders linked thereto determined by the test apparatus determination part from the identifying information of all test apparatuses and the identifying information of one or more test orders linked thereto, which are displayed by the first display image output part, in a different displaying form.

6. A computer program causing a computer to execute a test order processing to link each test result, sent from a test apparatus to the computer, to a corresponding test order, the computer program making the computer functions as:
a first display image output part that displays mutually linked one or more test orders and one or more test apparatuses on a display device in a displaying form showing which test order is linked to which test apparatus,
a test apparatus determination part that determines the test apparatus that should perform the test and one or more test orders linked thereto from the test orders and the test apparatuses linked thereto, which are displayed on the display device, by a user's input or by an automatic selection;
a test result receiving part that receives each test result sent from the test apparatus(es) connected to the test order processing apparatus; and
a (test result/test order) linking part that links one or more test results to the one or more test orders linked to the test apparatuses, according to the input sequence or the automatical selection sequence, the test results being received by the test result receiving part from the test apparatus that should perform the test, and the test apparatuses being determined by the test apparatus determination part.

7. The computer program according to claim 6, further comprising a program making the computer functions as:
a test order receiving part that receives the test order containing one or more test items from a sender of the test order;
a linking information-holding part that holds information linking said one or more test items contained in the test order to a test apparatus corresponding thereto; and
a (test order/test apparatus) linking part that refers to the linking information and test item(s) in the test order and links the test order to one or more corresponding test apparatuses.

8. The computer program according to claim 6 or 7, further comprising a program making the computer functions as a second display image output part that displays the test apparatus that should perform the test and one or more test orders linked thereto, which are determined by the test apparatus determination part, on the display device in a displaying form showing that they are determined.

9. The computer program according to any one of claims 6 to 8, further comprising a program making the computer function as a test report sending part that sends the test result, linked to the test order by the (test result/test order) linking part, to a given recipient as a test report or a part of a test report.
